# EUROPÄISCHE PATENTANMELDUNG

(11) **EP 2 995 599 A1**
(43) Veröffentlichungstag der Anmeldung: **16.03.2016**
(21) Anmeldenummer: 14184470.4
(22) Anmeldetag: 11.09.2014
(51) Int. Cl.: C07C 5/05, C07C 5/13

(54) **Verfahren und Anlage zur Herstellung von Kohlenwasserstoffen**

(71) Anmelder: Linde Aktiengesellschaft, 80331 München (DE)
(72) Erfinder: Dr. Höfel, Torben, 81543 München (DE); Fritz, Helmut, 81375 München (DE)
(74) Vertreter: m patent group

(57) **Zusammenfassung**

Es wird ein Verfahren zur Herstellung von Kohlenwasserstoffen vorgeschlagen, bei dem in einer Katalyseeinheit (1) unter Verwendung eines oder mehrerer, Oxygenate und/oder Olefine enthaltender Katalyseeinsatzströme (a) ein an n-Butan, Isobutan, 1-Buten, 2-Buten, Isobuten und Kohlenwasserstoffen mit mehr als vier und/oder weniger als drei Kohlenstoffatomen reicher Katalyseproduktstrom (b) erzeugt wird, und bei dem ferner in einer Dampfspalteinheit (2) unter Verwendung eines oder mehrerer Dampfspalteinsatzströme (g, r, s) ein Dampfspaltproduktstrom (h) erzeugt wird. Es ist vorgesehen, dass ein an 1-Buten, 2-Buten und Isobuten armer und zumindest Isobutan enthaltender Skelettisomerisierungseinsatzstrom (f, q) unter Verwendung des Katalyseproduktstroms (b) erzeugt wird, in dem das Isobutan zumindest zum überwiegenden Teil durch Skelettisomerisieren zu n-Butan umgesetzt wird, und der danach zumindest zum Teil als der oder einer der Dampfspalteinsatzströme (g, r) verwendet wird. Eine Anlage (100, 200) ist ebenfalls Gegenstand der Erfindung.

## Beschreibung

Die Erfindung betrifft ein Verfahren und eine Anlage zur Herstellung von Kohlenwasserstoffen gemäß den Oberbegriffen der jeweiligen unabhängigen Patentansprüche.

### Stand der Technik

Kurzkettige Olefine wie Ethylen und Propylen können durch Dampfspalten (engl. Steam Cracking) von Kohlenwasserstoffen hergestellt werden. Verfahren und Vorrichtungen zum Dampfspalten von Kohlenwasserstoffen sind beispielsweise im Artikel "Ethylene" in Ullmann's Encyclopedia of Industrial Chemistry, Onlineausgabe, 15. April 2007, DOI 10.1002/14356007.a10_045.pub2, beschrieben.

Ein alternativer Weg zu kurzkettigen Olefinen sind die sogenannten Oxygenat-zu-Olefin-Verfahren (engl. Oxygenates to Olefins, OTO). In Oxygenat-zu-Olefin-Verfahren werden Oxygenate wie Methanol oder Dimethylether in eine Reaktionszone eines Reaktors eingebracht, in der ein zur Umsetzung der Oxygenate geeigneter Katalysator bereitgestellt ist. Die Oxygenate werden beispielsweise zu Ethylen und Propylen umgesetzt. Die verwendeten Katalysatoren und Reaktionsbedingungen in Oxygenat-zu-Olefin-Verfahren sind dem Fachmann grundsätzlich bekannt.

Oxygenat-zu-Olefin-Verfahren können mit unterschiedlichen Katalysatoren durchgeführt werden. Beispielsweise können Zeolithe wie ZSM-5 oder SAPO-34 oder funktionell vergleichbare Materialien zum Einsatz kommen. Wenn ZSM-5 oder ein vergleichbares Material zum Einsatz kommt, bilden sich vergleichsweise große Mengen an längerkettigen (C3plus-)Kohlenwasserstoffen (zur Bezeichnung siehe unten) und eher geringe Mengen an kürzerkettigen (C2minus-)Kohlenwasserstoffen. Bei der Verwendung von SAPO-34 oder vergleichbaren Materialien werden im Gegensatz dazu eher kürzerkettige (C2minus-)Kohlenwasserstoffe gebildet.

Auch integrierte Verfahren und Anlagen ("Verbundanlagen") zur Herstellung von Kohlenwasserstoffen, die Dampfspaltverfahren und Oxygenat-zu-Olefin-Verfahren bzw. entsprechende Spaltöfen und Reaktoren umfassen, sind bekannt und beispielsweise in der WO 2011/057975 A2 oder der US 2013/0172627 A1 beschrieben.

Derartige integrierte Verfahren sind beispielsweise deshalb vorteilhaft, weil in den Oxygenat-zu-Olefin-Verfahren typischerweise nicht ausschließlich die gewünschten kurzkettigen Olefine gebildet werden. Ein wesentlicher Teil der Oxygenate wird in Paraffine und C4plus-Olefine umgewandelt. Gleichzeitig wird beim Dampfspalten nicht der gesamte Ofeneinsatz in kurzkettige Olefine gespalten. Im Spaltgas entsprechender Spaltöfen können insbesondere noch nicht umgesetzte Paraffine enthalten sein. Ferner finden sich hier typischerweise C4plus-Olefine, darunter Diolefine wie Butadien. Die erhaltenen Verbindungen richten sich in beiden Fällen nach den verwendeten Einsätzen und Reaktionsbedingungen.

In den in der WO 2011/057975 A2 und der US 2013/0172627 A1 vorgeschlagenen Verfahren werden das Spaltgas eines Spaltofens und der Abstrom eines Oxygenat-zu-Olefin-Reaktors in einer gemeinsamen Trenneinheit kombiniert und fraktioniert. Eine hier gewonnene C4-Fraktion kann, beispielsweise nach Hydrierung oder Abtrennung von Butadien, erneut einem Dampfspalt- und/oder einem Oxygenat-zu-Olefin-Verfahren unterworfen werden. Die C4-Fraktion kann in überwiegend olefinische und überwiegend paraffinische Teilfraktionen getrennt werden.

Die vorliegende Erfindung ist nicht auf Oxygenat-zu-Olefin-Verfahren beschränkt, sondern kann grundsätzlich mit beliebigen katalytischen Verfahren eingesetzt werden, insbesondere mit katalytischen Verfahren, bei denen die zuvor erläuterten Zeolithe als Katalysatoren zum Einsatz kommen. Als Einsatz in entsprechenden katalytischen Verfahren können neben Methanol und/oder Dimethylether auch andere Oxygenate verwendet werden, beispielsweise andere Alkohole und/oder Ether.

Ebenso können olefinische Komponenten, wie beispielsweise eine Mischung aus unterschiedlichen ungesättigten C4-Kohlenwasserstoffen, in entsprechenden katalytischen Verfahren eingesetzt werden. Man spricht in diesem Fall von einem Olefinspaltverfahren (engl. Olefins Cracking Process, OCP). Unterschiedliche Einsätze können im Rahmen der vorliegenden Erfindung in denselben Reaktor oder in unterschiedliche Reaktoren geführt werden. Beispielsweise kann in einem Reaktor ein Oxygenat-zu-Olefin-Verfahren und einem anderen Reaktor ein Olefinspaltverfahren durchgeführt werden. Beide Prozesse, aber auch ggf. ein kombinierter Prozess, haben jedoch zum Ziel, aus einem oder mehreren Einsätzen ein Produkt zu erzeugen, das reich an Propylen und ggf. Ethylen ist.

Die erläuterten katalytischen Verfahren, die sich insbesondere dadurch auszeichnen, dass dabei die erwähnten Zeolithe als Katalysatoren zum Einsatz kommen und ferner ein oder mehrere, Oxygenate und/oder Olefine enthaltende Katalyseeinsatzströme verwendet werden, werden damit in einer Katalyseeinheit durchgeführt, die einen oder mehrere entsprechender Reaktoren aufweisen kann.

Wie bereits erwähnt, ist Ziel entsprechender katalytischer Verfahren, Produkte zu erzeugen, die reich an Propylen und ggf. Ethylen sind. Typischerweise werden in entsprechenden Verfahren aber auch signifikante Mengen von Kohlenwasserstoffen mit vier und mehr Kohlenstoffatomen erzeugt. Aus dem Stand der Technik ist daher bekannt, entsprechende Kohlenwasserstoffe mit vier oder mehr Kohlenstoffatomen zur Katalyse zu rezyklieren. Auch eine Entnahme entsprechender Kohlenwasserstoffe als Produkt(e) ist bekannt. Ferner wurde bereits vorgeschlagen, die Kohlenwasserstoffe mit vier und mehr Kohlenwasserstoffatomen oder Teile bzw. Fraktionen hier von weiteren Reaktionsprozessen zuzuführen.

Sämtliche der bekannten Verfahren weisen jedoch Nachteile auf. Insbesondere ist die Effizienz der Verwertung entsprechender Kohlenwasserstoffe in solchen Verfahren häufig nicht zufriedenstellend.

Es besteht daher der Bedarf nach Verbesserungen bei entsprechenden Verfahren und Anlagen zur Herstellung von Kohlenwasserstoffen unter Verwendung der erläuterten katalytischen Verfahren.

### Offenbarung der Erfindung

Diese Aufgabe wird durch ein Verfahren und eine Anlage zur Herstellung von Kohlenwasserstoffen mit den Merkmalen der unabhängigen Patentansprüche gelöst. Bevorzugte Ausgestaltungen sind Gegenstand der abhängigen Patentansprüche sowie der nachfolgenden Beschreibung.

Vor der detaillierten Erläuterung der Merkmale und Vorteile der vorliegenden Erfindung werden deren Grundlagen und die verwendeten Begriffe erläutert.

Flüssige und gasförmige Ströme können im hier verwendeten Sprachgebrauch reich oder arm an einer oder mehreren Komponenten sein, wobei "reich" für einen Gehalt von wenigstens 75%, 90%, 95%, 99%, 99,5%, 99,9% oder 99,99% und "arm" für einen Gehalt von höchstens 25%, 10%, 5%, 1%, 0,1% oder 0,01% auf molarer, Gewichts- oder Volumenbasis stehen kann. Der Begriff "überwiegend" kann der soeben getroffenen Definition von "reich" entsprechen, bezeichnet jedoch insbesondere einen Gehalt oder Anteil von mehr als 90%. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Ein flüssiger oder gasförmiger Strom ist von einem anderen flüssigen oder gasförmigen Strom (auch als Ausgangsstrom bezeichnet) "abgeleitet" oder aus diesem "gebildet", wenn er zumindest einige in dem Ausgangsstrom enthaltene oder aus diesem erhaltene Komponenten aufweist. Ein in diesem Sinne abgeleiteter oder gebildeter Strom kann aus dem Ausgangsstrom insbesondere durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden.

Gängige Verfahren zur Trennung von Produktströmen aus Verfahren zur Herstellung von Kohlenwasserstoffen umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem und zwei Kohlenstoffatomen.

Unter Oxygenaten werden typischerweise Ether und Alkohole verstanden. Neben Methyl-*tert*-butylether (MTBE, engl. methyl tertiary butyl ether) kommen beispielsweise *tert-*Amylmethylether (TAME, tertiary amyl methyl ether), *tert*-Amylethylether (TAEE, tertiary amyl ethyl ether), Ethyl-*tert*-butylether (ETBE, ethyl tertiary butyl ether) und Diisopropylether (DIPE, diisopropyl ether) zum Einsatz. Als Alkohole werden beispielsweise Methanol, Ethanol und *tert*-Butanol (TBA, tertiary butyl alcohol) verwendet. Zu den Oxygenaten zählt insbesondere auch Dimethylether (DME, dimethyl ether). Die Erfindung eignet sich auch zur Verwendung mit anderen Oxygenaten.

Gemäß einer gängigen Definition, die auch hier Anwendung findet, handelt es sich bei Oxygenaten um Verbindungen, die wenigstens eine kovalent an ein Sauerstoffatom gebundene Alkylgruppe aufweisen. Die wenigstens eine Alkylgruppe kann bis zu fünf, bis zu vier oder bis zu drei Kohlenstoffatome aufweisen. Insbesondere weisen die Oxygenate, die im Rahmen der vorliegenden Erfindung von Interesse sind, Alkylgruppen mit einem oder zwei Kohlenstoffatomen auf, insbesondere handelt es sich um Methylgruppen. Insbesondere handelt es sich um einwertige Alkohole und Dialkylether wie Methanol und Dimethylether oder entsprechende Mischungen.

Dampfspaltverfahren werden im kommerziellen Maßstab nahezu ausschließlich in Rohrreaktoren durchgeführt, in denen einzelne Reaktionsrohre (in Form von Rohrschlangen, sogenannten Coils) oder Gruppen von entsprechenden Reaktionsrohren auch bei unterschiedlichen Spaltbedingungen betrieben werden können. Unter gleichen oder vergleichbaren Spaltbedingungen betriebene Reaktionsrohre oder Gruppen von Reaktionsrohren, gegebenenfalls aber auch unter einheitlichen Spaltbedingungen betriebene Rohrreaktoren insgesamt, werden auch jeweils als "Spaltöfen" bezeichnet. Ein Spaltofen ist im hier verwendeten Sprachgebrauch also eine zum Dampfspalten verwendete bauliche Einheit, die einen Ofeneinsatz gleichen oder vergleichbaren Spaltbedingungen aussetzt. Eine im Rahmen der vorliegenden Erfindung eingesetzte Dampfspalteinheit kann einen oder mehrere derartiger Spaltöfen aufweisen.

Entsprechendes gilt auch, wie bereits angesprochen, für die im Rahmen der vorliegenden Erfindung verwendete Katalyseeinheit, in der unterschiedliche Reaktoren mit gleichen oder unterschiedlichen Katalysatoren ausgestattet, mit gleichen oder unterschiedlichen Einsatzströmen beschickt und bei gleichen oder unterschiedlichen Reaktionsbedingungen betrieben werden können.

Mit dem Begriff "Dampfspalteinsatzströme" werden hier ein oder mehrere flüssige und/oder gasförmige Ströme bezeichnet, die einem oder mehreren Spaltöfen zugeführt werden. Auch durch ein entsprechendes Dampfspaltverfahren erhaltene Ströme, wie unten erläutert, können in einen oder mehrere Spaltöfen zurückgeführt und damit erneut als Dampfspalteinsatzströme verwendet werden. Als Dampfspalteinsatzströme eignen sich eine Vielzahl von Kohlenwasserstoffen und Kohlenwasserstoffgemischen von Ethan bis Gasöl bis zu einem Siedepunkt von typischerweise 600 °C.

Ein Dampfspalteinsatzstrom kann also ausschließlich sogenannten "Frischeinsatz" umfassen, also einen Einsatz, der anlagenextern bereitgestellt und beispielsweise aus einer oder mehreren Erdölfraktionen, Erdgas und/oder Erdgaskondensaten gewonnen wird. Ein Dampfspalteinsatzstrom kann aber auch zusätzlich oder ausschließlich einen oder mehrere sogenannte "Recycleströme" umfassen, also Ströme, die in der Anlage selbst erzeugt und in einen entsprechenden Spaltofen zurückgeführt werden. Ein Dampfspalteinsatzstrom kann auch aus einem Gemisch eines oder mehrerer Frischeinsätze mit einem oder mehreren Recycleströmen bestehen.

Der Dampfspalteinsatzstrom wird im jeweiligen Spaltofen zumindest teilweise umgesetzt und verlässt den Spaltofen als sogenanntes "Rohgas", das Nachbehandlungsschritten unterworfen werden kann. Diese Nachbehandlungsschritte umfassen zunächst eine Aufbereitung des Rohgases, beispielsweise durch Quenchen, Kühlen und Trocknen, wodurch ein "Spaltgas" erhalten wird. Bisweilen wird auch bereits das Rohgas als Spaltgas bezeichnet. Im vorliegenden Fall wird hierfür der Begriff "Dampfspaltproduktstrom" verwendet.

Wiederum gilt Entsprechendes auch für den oder die einer oder mehreren Katalyseeinheiten zugeführten Einsatzströme, die hier als "Katalyseeinsatzströme" bezeichnet werden. Der oder die Katalyseeinsatzströme werden in der Katalyseeinheit in einem oder mehreren Reaktoren zu einem oder mehreren Produktströmen, die hier als "Katalyseproduktströme" bezeichnet werden, umgesetzt.

In neueren Verfahren und Vorrichtungen zum Dampfspalten kommen zunehmend milde Spaltbedingungen zum Einsatz, weil sich bei diesen insbesondere sogenannte Wertprodukte, beispielsweise Propylen, in größerer Menge gewinnen lassen. Grundsätzlich sind Verfahren von Vorteil, bei denen die Spaltbedingungen an die Zusammensetzung des oder der Dampfspalteinsatzströme angepasst werden. Bei milden Spaltbedingungen reduziert sich jedoch auch die Umsetzung in dem oder den Spaltöfen, so dass sich nicht umgesetzte Verbindungen in vergleichsweise großer Menge in dem oder den Dampfspaltproduktströmen wiederfinden und auf diese Weise zu einer "Verdünnung" der zu gewinnenden Wertprodukte führen.

Die erwähnten "Spaltbedingungen" in einem Spaltofen umfassen unter anderem den Partialdruck des Ofeneinsatzes, der durch die Zugabe unterschiedlicher Dampfmengen und den im Spaltofen eingestellten Druck beeinflusst werden kann, die Verweildauer im Spaltofen sowie die in diesem verwendeten Temperaturen und Temperaturprofile. Auch die Ofengeometrie und Ofengestaltung spielt eine Rolle.

Da die genannten Werte einander zumindest teilweise wechselseitig beeinflussen, hat sich zur Charakterisierung der Spaltbedingungen der Begriff der "Spaltschärfe" (engl. Cracking Severity) durchgesetzt. Für flüssige Ofeneinsätze kann die Spaltschärfe über das Verhältnis von Propylen zu Ethylen (P/E) oder als das Verhältnis von Methan zu Propylen (M/P) im Spaltgas auf Gewichtsbasis (kg/kg) beschrieben werden. Für gasförmige Ofeneinsätze kann dagegen die Umsetzung bzw. Konversion einer jeweils betrachteten Komponente des Ofeneinsatzes als Maß der Spaltschärfe angegeben werden. Insbesondere für Kohlenwasserstoffe mit vier Kohlenstoffatomen ist eine Beschreibung der Spaltschärfe über die Umsetzung von Schlüsselkomponenten wie *n-*Butan und Isobutan gut geeignet. Zum fachmännischen Verständnis des Begriffs der Spaltschärfe (engl. Severity) sei beispielsweise auf den erwähnten Artikel "Ethylene" in Ullmann's Encyclopedia of Industrial Chemistry verwiesen.

### Vorteile der Erfindung

Die vorliegende Erfindung kombiniert die eingangs erläuterten Maßnahmen zur Nutzung von Kohlenwasserstoffen mit vier Kohlenstoffatomen aus einem entsprechenden Katalyseverfahren in optimierter Weise, so dass insgesamt eine effiziente Verwertung mit maximaler Wertschöpfung und geringen internen Recycleströmen erfolgt.

Zu diesem Zweck geht die vorliegende Erfindung von einem Verfahren zur Herstellung von Kohlenwasserstoffen aus, das umfasst, in einer Katalyseeinheit unter Verwendung eines oder mehrerer, Oxygenate und/oder Olefine enthaltender Katalyseeinsatzströme einen n-Butan, Isobutan, 1-Buten, 2-Buten, Isobuten und Kohlenwasserstoffe mit mehr als vier und/oder weniger als drei Kohlenstoffatomen enthaltenden Katalyseproduktstrom zu erzeugen. Die Katalyseeinheit umfasst dabei, wie zuvor erläutert, einen oder mehrere Reaktoren, die mit einem oder mehreren Einsatzströmen, die hier als Katalyseeinsatzströme bezeichnet werden, beschickt werden.

Wie erläutert, eignet sich die vorliegende Erfindung zur Verwendung mit Oxygenat-zu-Olefin-Verfahren und/oder den sogenannten Olefinspaltverfahren und anderen Verfahren. Der oder die in einer entsprechenden Katalyseeinheit verwendeten Reaktoren weisen vorzugsweise Zeolithe als Katalysatoren auf. Wie erläutert, können diese Katalysatoren insbesondere vom SAPO- oder ZSM-Typ sein. Die im Rahmen der vorliegenden Erfindung eingesetzte Katalyseeinheit ist damit für ein entsprechendes Katalyseverfahren eingerichtet.

Ferner sieht das Verfahren vor, in einer Dampfspalteinheit unter Verwendung eines oder mehrerer Dampfspalteinsatzströme einen Dampfspaltproduktstrom zu erzeugen. Das im Rahmen der vorliegenden Erfindung eingesetzte Dampfspaltverfahren kann dabei unter Verwendung gleicher oder unterschiedlicher Dampfspaltbedingungen in einem oder mehreren Spaltöfen erfolgen, wie grundsätzlich bekannt. Zu Details wird auf die obigen Erläuterungen verwiesen. Insbesondere können die beim Dampfspalten eingesetzten Dampfspalteinsatzströme mild gespalten werden, um die Ausbeute an Wertprodukten zu erhöhen. Schärfere Spaltbedingungen können insbesondere eingesetzt werden, um einen möglichst hohen Umsatz zu erzielen.

Erfindungsgemäß ist nun vorgesehen, dass unter Verwendung des Katalyseproduktstroms ein an 1-Buten, 2-Buten und Isobuten armer und zumindest Isobutan enthaltender Skelettisomerisierungseinsatzstrom erzeugt wird, in welchem das Isobutan zumindest zum überwiegenden Teil durch Skelettisomerisieren zu *n-*Butan umgesetzt wird, und der danach zumindest zum Teil als der oder einer der Dampfspalteinsatzströme verwendet wird.

Ein wesentlicher Aspekt der vorliegenden Erfindung ist es also, eine Skelettisomerisierung zur Aufbereitung von verzweigten Kohlenwasserstoffen mit vier Kohlenstoffatomen aus einem Dampfspaltverfahren zu verwenden. Im Ergebnis werden überwiegend unverzweigte Komponenten dem Dampfspaltverfahren unterworfen, was einen erhöhten Umsatz sowie eine verbesserte Selektivität in Richtung der erwünschten Zielprodukte Ethylen und Propylen zur Folge hat.

Wie auch nachfolgend noch erläutert, kann der an 1-Buten, 2-Buten und Isobuten arme und zumindest Isobutan enthaltende Skelettisomerisierungseinsatzstrom auch dadurch erzeugt werden, dass, unter anderem, in den Dampfspaltproduktstrom noch enthaltenes Isobuten durch Hydrieren zu Isobutan umgesetzt wird. Dieses kann anschließend in dem Skelettisomerisierungseinsatzstrom der Skelettisomerisierung unterworfen werden. Insbesondere Isobuten ist aufgrund seiner thermischen Stabilität ausgesprochen ungeeignet zum Einsatz in einem Dampfspaltverfahren und kann daher in herkömmlichen Verfahren dort nur schlecht genutzt werden. Ein entsprechender Skelettisomerisierungseinsatzstrom kann dadurch gewonnen werden, dass unter anderem sämtliche enthaltenen Olefine hydriert werden. Wahlweise ist es auch möglich, durch eine Destillation, der optional eine Hydroisomerisierung von 1-Buten zu 2-Buten vorangeht, den Skelettisomerisierungseinsatzstrom zu gewinnen.

Ist hier und im Folgenden von einer Entfernung, Bearbeitung, Abtrennung oder Umsetzung "zumindest des überwiegenden Teils" eines oder mehrerer Kohlenwasserstoffe die Rede, kann hiervon umfasst sein, dass, wie einleitend erläutert, mindestens 75%, 90% oder mehr solcher Kohlenwasserstoffe entfernt werden. Vorzugsweise werden entsprechende Kohlenwasserstoffe im Wesentlichen vollständig entfernt, also insbesondere zu mindestens 95%, gegebenenfalls auch zu wenigstens 99% oder mehr.

Das im Rahmen der vorliegenden Erfindung vorgeschlagene Verfahren ist besonders effizient, da hier auch das für die Dampfspaltung eigentlich ungeeignete Isobuten in Form von Isobutan, das anschließend zu n-Butan umgesetzt wird, genutzt wird, und damit wie alle anderen Komponenten als Dampfspalteinsatzstrom bzw. Dampfspaltströme der Dampfspaltung zugeführt werden kann. Auf diese Weise ist es auch möglich, die Produktmenge von Butadien, die in der Dampfspaltung gebildet wird, zu maximieren, da die Spaltbedingungen bei Abwesenheit von Isobuten weitgehend in Richtung einer maximalen Butadienproduktion angepasst werden können.

Ein wesentlicher Unterschied im Rahmen der vorliegenden Erfindung gegenüber einem bekannten Verfahren, wie es beispielsweise in der US 2013/0172627 A1 offenbart ist, besteht darin, dass im Rahmen der vorliegenden Erfindung paraffinische und olefinische Komponenten, inklusive Isobuten nach entsprechender Umsetzung, zum Dampfspalten geführt werden können und nicht lediglich eine Trennung in C4-Olefine und C4-Paraffine erfolgt.

Nachfolgend werden bereits teilweise erläuterte Ausführungsformen der Erfindung, wie sie in den abhängigen Patentansprüchen wiedergegeben sind, nochmals zusammengefasst erläutert.

Insbesondere sieht das erfindungsgemäße Verfahren vor, unter Verwendung zumindest eines Teils des Katalyseproduktstroms einen Trenneinsatzstrom zu bilden, aus dem die Kohlenwasserstoffe mit mehr als vier und/oder weniger als drei Kohlenstoffatomen unter Erhalt eines an n-Butan, Isobutan, 1-Buten, 2-Buten und Isobuten reichen Trennabstroms zumindest zum überwiegenden Teil abgetrennt werden. Einem entsprechenden Trenneinsatzstrom kann neben dem Katalyseproduktstrom oder einem Teil hiervon grundsätzlich auch ein weiterer Strom zugespeist werden, beispielsweise der oder zumindest ein Teil des Dampfspaltproduktstroms, der auf diese Weise in derselben Trenneinrichtung bearbeitet werden kann, wie der Katalyseproduktstrom oder ein entsprechender Teil hiervon. Hierdurch ergibt sich nochmals eine verbesserte Integration eines katalytischen Verfahrens, wie zuvor erläutert, und eines Dampfspaltverfahrens. Die aus dem Trenneinsatzstrom zumindest zum überwiegenden Teil abgetrennten Kohlenwasserstoffe mit mehr als vier und/oder weniger als drei Kohlenstoffatomen können in Form einzelner Fraktionen nochmals aufgetrennt, als Produkte bereitgestellt und/oder in die Katalyseeinheit und/oder die Dampfspalteinheit zurückgeführt werden. Details sind der Übersichtlichkeit halber hier nicht erläutert.

Mit besonderem Vorteil umfasst ein erfindungsgemäßes Verfahren, dass unter Verwendung zumindest eines Teils des Trennabstroms, der, wie erwähnt, an n-Butan, Isobutan, 1-Buten, 2-Buten und Isobuten reich ist und wenige oder keine Kohlenwasserstoffe mit mehr als vier und/oder weniger als drei Kohlenstoffatomen enthält, einen Hydrierungseinsatzstrom zu bilden, in welchem das 1-Buten, 2-Buten und Isobuten unter Erhalt eines Hydrierungsabstroms zumindest zum überwiegenden Teil durch Hydrieren zu n-Butan und Isobutan umgesetzt werden. Ist hier und im Folgenden davon die Rede, dass ein entsprechender Strom "gebildet" wird, kann dies auch lediglich das Verwenden eines entsprechenden Stroms umfassen, der nicht notwendigerweise bearbeitet werden muss, wie oben erläutert. Zumindest ein Teil des erhaltenen Hydrierungsabstroms kann bei der Bildung des Skelettisomerisierungseinsatzstroms verwendet werden. Wie soeben erläutert, kann also der gesamte oder nur ein Teil des Hydrierungsabstroms als Skelettisomerisierungseinsatzstrom verwendet werden.

Besonders vorteilhaft ist es, wenn unter Verwendung zumindest eines Teils des Trennabstroms ein Destillationseinsatzstrom gebildet wird, aus dem das n-Butan und 2-Buten unter Erhalt eines an n-Butan und 2-Buten armen Destillationsabstroms zumindest zum überwiegenden Teil abgetrennt werden. Eine entsprechende destillative Abtrennung erleichtert die anschließende Bearbeitung des Destillationsabstroms, aus dem, wie nachfolgend erläutert, oder unter dessen Verwendung der Skelettisomerisierungseinsatzstrom erzeugt wird. Bei der Skelettisomerisierung bzw. in dieser vorangehenden Schritten müssen aufgrund einer entsprechenden Destillation deutlich geringere Volumenströme bewältigt werden.

Ein besonders vorteilhaftes Verfahren, bei dem eine entsprechende Destillation eingesetzt wird, umfasst, unter Verwendung zumindest eines Teils des Trennabstroms einen Hydroisomerisierungseinsatzstrom zu bilden, in welchem das 1-Buten unter Erhalt eines Hydroisomerisierungsabstroms zumindest zum überwiegenden Teil durch Hydroisomerisieren zu 2-Buten umgesetzt wird. Zumindest ein Teil des Hydroisomerisierungsabstroms kann dann bei der Bildung des Destillationseinsatzstroms verwendet werden. Eine entsprechende Hydroisomerisierung erleichtert die Trennung von Isobuten, das anschließend, wie erwähnt, zur Hydrierung und Skelettisomerisierung geführt wird, von den linearen Butenen, die nicht notwendigerweise einer entsprechenden Behandlung unterworfen werden müssen, beträchtlich: Der Siedepunkt von Isobuten bei Atmosphärendruck liegt bei -6,9 °C, jener von 1-Buten bei -6,47 °C. Eine destillative Trennung ist damit praktisch nicht möglich. Der Siedepunkt der 2-Butene liegt hingegen deutlich oberhalb hiervon, nämlich bei 3,7 °C für cis-2-Buten und bei 0,9 °C für *trans*-2-Buten.

Unter Verwendung zumindest eines Teils des Destillationsabstroms eines zuvor erläuterten Destillationsverfahrens wird vorteilhafterweise ein Hydrierungseinsatzstrom gebildet, in dem zumindest das Isobutan unter Erhalt eines Hydrierungsabstroms zumindest zum überwiegenden Teil durch Hydrieren zu Isobutan umgesetzt wird. Der erhaltene Hydrierungsabstrom wird zumindest zum Teil bei der Bildung des Skelettisomerisierungseinsatzstroms verwendet, in welchem das durch die Hydrierung aus dem Isobuten erhaltene Isobutan durch Skelettisomerisierung besonders einfach umgesetzt werden kann.

Wird ein destillatives Verfahren verwendet, wie zuvor erläutert, wird vorteilhafterweise unter Verwendung zumindest eines Teils des aus dem Destillationseinsatzstrom abgetrennten n-Butans und 2-Butens ein Strom gebildet, der als weiterer Dampfspalteinsatzstrom verwendet werden kann. Der weitere Dampfspalteinsatzstrom kann in denselben oder einen anderen Spaltofen als der zuvor erläuterte, aus dem Skelettisomerisierungseinsatzstrom oder unter Verwendung desselben gebildete Dampfspalteinsatzstrom geführt werden.

In dem Dampfspaltverfahren bzw. der Dampfspalteinheit wird ein Dampfspaltproduktstrom gebildet, der Kohlenwasserstoffe mit vier Kohlenstoffatomen, darunter Butadien, sowie Kohlenwasserstoffe mit mehr als vier und/oder weniger als drei Kohlenstoffatomen enthält. Wie erläutert, kann ein entsprechender Strom auch in einer Trenneinheit, die der Katalyseeinheit zugeordnet ist, bearbeitet werden. Vorteilhafterweise wird unter Verwendung zumindest eines Teils des Dampfspaltproduktstroms ein an Butadien und Kohlenwasserstoffen mit mehr als vier und/oder weniger als drei Kohlenstoffatomen armer Reststrom gewonnen, der zumindest zum Teil bei der Erzeugung des Skelettisomerisierungseinsatzstroms verwendet wird. Insbesondere kann ein entsprechender Reststrom beispielsweise einer Hydrierung vor einer destillativen Trennung oder einer Hydroisomerisierung unterworfen werden.

Eine Anlage, die zur Herstellung von Kohlenwasserstoffen eingerichtet ist, ist ebenfalls Gegenstand der Erfindung. Eine entsprechende Anlage weist eine Katalyseeinheit auf, die dazu eingerichtet ist, unter Verwendung eines oder mehrerer, Oxygenate und/oder Olefine enthaltender Katalyseeinsatzströme einen an n-Butan, Isobutan, 1-Buten, 2-Buten, Isobuten und Kohlenwasserstoffen mit mehr als vier und/oder weniger als drei Kohlenstoffatomen reichen Katalyseproduktstrom zu erzeugen, sowie eine Dampfspalteinheit, die dazu eingerichtet ist, unter Verwendung eines oder mehrerer Dampfspalteinsatzströme einen Dampfspaltproduktstrom zu erzeugen.

Eine entsprechende Anlage zeichnet sich durch Mittel aus, die dazu eingerichtet sind, unter Verwendung des Katalyseproduktstroms einen an 1-Buten, 2-Buten und Isobuten armen und zumindest Isobutan enthaltenden Skelettisomerisierungseinsatzstrom zu erzeugen, in diesem das Isobutan zumindest zum überwiegenden Teil durch Skelettisomerisierung zu n-Butan umzusetzen, und diesen danach zumindest zum Teil als den oder einen der Dampfspalteinsatzströme zu verwenden.

Eine entsprechende Anlage, die insbesondere Mittel aufweist, die zur Durchführung eines Verfahrens eingerichtet sind, wie es zuvor erläutert wurde, profitiert von den zuvor erläuterten Vorteilen, auf die daher ausdrücklich verwiesen wird.

Verfahren zur Hydrierung, Skelettisomerisierung und Hydroisomerisierung, wie sie im Rahmen der vorliegenden Erfindung zum Einsatz kommen, sind dem Fachmann grundsätzlich bekannt.

Eine Skelettisomerisierung kann beispielsweise unter Verwendung von Aluminiumoxidkatalysatoren (in denen y-Aluminiumoxid als Adsorbens, als Katalysatorträger und/oder als Katalysator selbst Verwendung finden kann) vorgenommen werden. Hierbei können auch beispielsweise aktiviertes und/oder dampfbehandeltes Aluminiumoxid zum Einsatz kommen, wie in der US 3 558 733 A angegeben. Ferner können titan- oder borhaltige Verbindungen verwendet werden, insbesondere in Verbindung mit η- oder y-Aluminiumoxid, wie in der US 5 321 195 A und der US 5 659 104 A beschrieben. Weitere einsetzbare Verbindungen sind halogenierte Aluminiumoxide, wie beispielsweise in der US 2 417 647 A offenbart, Bauxite oder Zeolithe. Auch die Verwendung von mikroporös strukturierten Molekularsieben ist, beispielsweise aus der EP 0 523 838 A1, der EP 0 501 577 A1 sowie der EP 0 740 957 A1, bekannt. Letztere können auch aktive Phasen von Katalysatoren bilden. Aluminiumoxidbasierte Katalysatoren werden im Allgemeinen in Anwesenheit von Wasser bei Temperaturen von 200 bis 700 °C und Drücken von 0,1 bis 2 MPa, insbesondere bei Temperaturen von 300 bis 570 °C und Drücken von 0,1 bis 1 MPa, eingesetzt. Weitere Reaktionsbedingungen zur Skelettisomerisierung können den genannten Druckschriften entnommen werden.

Zur Hydrierung und Hydroisomerisierung von Olefinen oder olefinhaltigen Kohlenwasserstoffgemischen sind aus dem Stand der Technik zahlreiche katalytische Verfahren bekannt, die auch im Rahmen der vorliegenden Erfindung zum Einsatz kommen können. Hydrierkatalysatoren besitzen als hydrieraktive Komponente ein oder mehrere Elemente der 6., 7. oder 8. Nebengruppe des Periodensystems in elementarer oder gebundener Form. Als Hydroisomerisierungskatalysatoren werden typischerweise Edelmetalle der 8. Nebengruppe in elementarer Form eingesetzt. Sie können mit unterschiedlichen Zusätzen dotiert sein, um bestimmte Katalysatoreigenschaften, beispielsweise Lebensdauer, Resistenz gegen bestimmte Katalysatorgifte, Selektivität oder Regenerierbarkeit zu beeinflussen. Die Hydrier- und Hydroisomerisierungskatalysatoren enthalten die aktive Komponente vielfach auf Trägern, beispielsweise Mordeniten, Zeolithen, Al₂O₃-Modifikationen, SiO₂-Modifikationen und anderen.

Hydroisomerisierungsverfahren sind beispielsweise in der EP 1 871 730 B1, der US 2002/169346 A1, der US 6 420 619 B1, der US 6 075 173 A und der WO 93/21137 A1 beschrieben. Bei solchen Verfahren wird typischerweise ein entsprechender Strom in Anwesenheit eines Hydroisomerisierungskatalysators durch einen Hydroisomerisierungsreaktor geführt. Der Hydroisomerisierungsreaktor ist typischerweise als Festbettreaktor ausgebildet. Vorzugsweise wird mittels des Hydroisomerisierungsverfahrens eine weitestgehende Umsetzung des 1-Butens zu 2-Buten erzielt. Die tatsächlich vorgenommene Umsetzung ergibt sich jedoch unter anderem aus Wirtschaftlichkeitserwägungen.

Zur weitgehenden Hydrierung der Olefine werden im Allgemeinen Reaktionstemperaturen von 150 bis 250 °C angewandt, die Hydroisomerisierung erfolgt bei deutlich geringeren Temperaturen. Das thermodynamische Gleichgewicht liegt bei diesen niedrigeren Temperaturen auf Seiten der internen Olefine, hier 2-Buten.

Die Erfindung und bevorzugte Ausführungsformen der Erfindung werden nachfolgend unter Bezugnahme auf die beigefügten Zeichnungen näher erläutert, die bevorzugte Ausführungsformen der Erfindung zeigen.

### Kurze Beschreibung der Zeichnungen

Figur 1 zeigt eine Anlage gemäß einer Ausführungsform der Erfindung in schematischer Darstellung.
Figur 2 zeigt eine Anlage gemäß einer Ausführungsform der Erfindung in schematischer Darstellung.

### Ausführungsformen der Erfindung

In den Figuren sind einander entsprechende Elemente mit identischen Bezugszeichen angegeben und werden der Übersichtlichkeit halber nicht wiederholt erläutert. Die in den jeweiligen Figuren gezeigten Ströme sind dann mit identischen Bezugszeichen versehen, wenn sie im Wesentlichen gleiche oder vergleichbare Zusammensetzung aufweisen, ungeachtet möglicherweise unterschiedlicher Volumenströme. In allen Figuren ist eine Katalyseeinheit mit 1 und eine Dampfspalteinheit mit 2 bezeichnet.

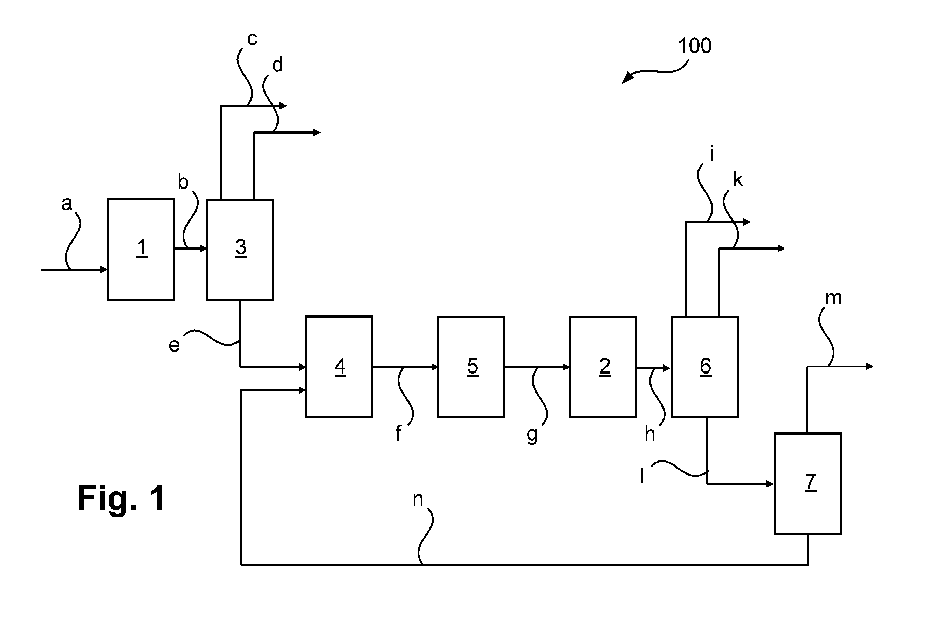

In Figur 1 ist eine Anlage gemäß einer Ausführungsform der Erfindung vereinfacht schematisch dargestellt und insgesamt mit 100 bezeichnet.

Der Katalyseeinheit 1 werden hier ein oder mehrere, Oxygenate und/oder Olefine enthaltende Katalyseeinsatzströme, hier mit a bezeichnet, zugeführt. Wie erwähnt, kann die Katalyseeinheit 1 einen oder mehrere Reaktoren umfassen, die mit einem Zeolithkatalysator betrieben werden. Die Katalyseeinheit kann zusätzlich mit weiteren Strömen, wie hier nicht veranschaulicht, beschickt werden.

In der Katalyseeinheit 1 wird im dargestellten Beispiel ein Katalyseproduktstrom b erzeugt. Dieser wird als Trenneinsatzstrom einer Trenneinheit 3 zugeführt, in der aus dem Katalyseproduktstrom b ein an Kohlenwasserstoffen mit mehr als vier und/oder weniger als drei Kohlenstoffatomen abgereicherter bzw. an Kohlenwasserstoffen mit vier Kohlenstoffatomen reicher Strom e, hier als Trennabstrom bezeichnet, erhalten wird. Die dabei abgetrennten Ströme, hier mit c und d bezeichnet, können beispielsweise Kohlenwasserstoffe mit fünf und mehr und/oder Kohlenwasserstoffe mit drei und weniger Kohlenstoffatomen oder entsprechende andere Fraktionen umfassen. Solche Ströme können ebenfalls in einer entsprechenden Anlage bearbeitet und/oder als Produkte gewonnen werden.

Der Trennabstrom e wird im dargestellten Beispiel mit einem weiteren, unten erläuterten Strom n, hier als Reststrom bezeichnet, vereinigt, wodurch ein Hydrierungseinsatzstrom erzeugt wird, der einer Hydriereinheit 4 zugeführt wird. In der Hydriereinheit 4 werden vorzugsweise sämtliche ungesättigten Kohlenwasserstoffe der Ströme e und n zu entsprechenden gesättigten Kohlenwasserstoffen umgesetzt. Auch eine nur teilweise Hydrierung ist jedoch möglich. In der Hydriereinheit 4 wird ein Strom gewonnen, der hier als Hydrierungsabstrom bezeichnet wird.

Während der Trennabstrom e im dargestellten Beispiel typischerweise noch sämtliche Kohlenwasserstoffe mit vier Kohlenstoffatomen, die in der Katalyseeinheit 1 erzeugt werden, oder aus dem oder den Katalyseeinsatzströmen a stammen und in der Katalyseeinheit 1 nicht umgesetzt wurden, enthält, insbesondere n-Butan, Isobutan, 1-Buten, 2-Buten und Isobuten, enthält der Hydrierungsabstrom nur noch oder überwiegend die entsprechenden gesättigten Kohlenwasserstoffe, d. h. n-Butan und Isobutan.

Im dargestellten Beispiel wird der Hydrierungsabstrom als Skelettisomerisierungseinsatzstrom f einer Isomerisierungseinheit 5 zugeführt, in welcher das in dem Skelettisomerisierungseinsatzstrom f enthaltene Isobutan zu *n-*Butan umgesetzt wird. Ein in der Skelettisomerisierungseinheit 5 erhaltener Skelettisomerisierungsabstrom enthält daher überwiegend oder ausschließlich n-Butan und wird als Dampfspalteinsatzstrom g der Dampfspalteinheit 2 zugeführt.

Der Dampfspalteinsatzstrom g, d.h. beispielsweise im Wesentlichen reines n-Butan, wird in der Dampfspalteinheit 2 in einem oder mehreren Spaltöfen, gegebenenfalls auch zusammen mit weiteren Strömen, die in gleiche oder unterschiedliche Spaltöfen geführt werden, bearbeitet. Es wird ein Dampfspaltproduktstrom h erhalten, der, wie erläutert, Kohlenwasserstoffe mit vier Kohlenstoffatomen, darunter Butadien, sowie Kohlenwasserstoffe mit mehr als vier und/oder weniger als drei Kohlenstoffatomen enthält. Dieser Dampfspaltproduktstrom h wird einer weiteren Trenneinheit 6 zugeführt, in der zunächst unter Abtrennung von Kohlenwasserstoffen mit mehr als vier und/oder weniger als drei Kohlenstoffatomen, wie hier mit den Strömen i und k veranschaulicht, ein Strom I erhalten wird, der überwiegend Kohlenwasserstoffe mit vier Kohlenstoffatomen, darunter Butadien, enthält. Der Strom I wird im dargestellten Beispiel in eine Butadienabtrenneinheit 7 geführt, wo das enthaltende Butadien zu einem überwiegenden Anteil abgetrennt und als Strom m aus der Anlage ausgeführt wird. Ein verbleibender, butadienarmer Reststrom, hier mit n bezeichnet, kann mit dem zuvor erläuterten Hydrierungseinsatzstrom bzw. dem diesen bildenden Trennabstrom e vereinigt und in die Hydrierungseinheit 4 geführt werden.

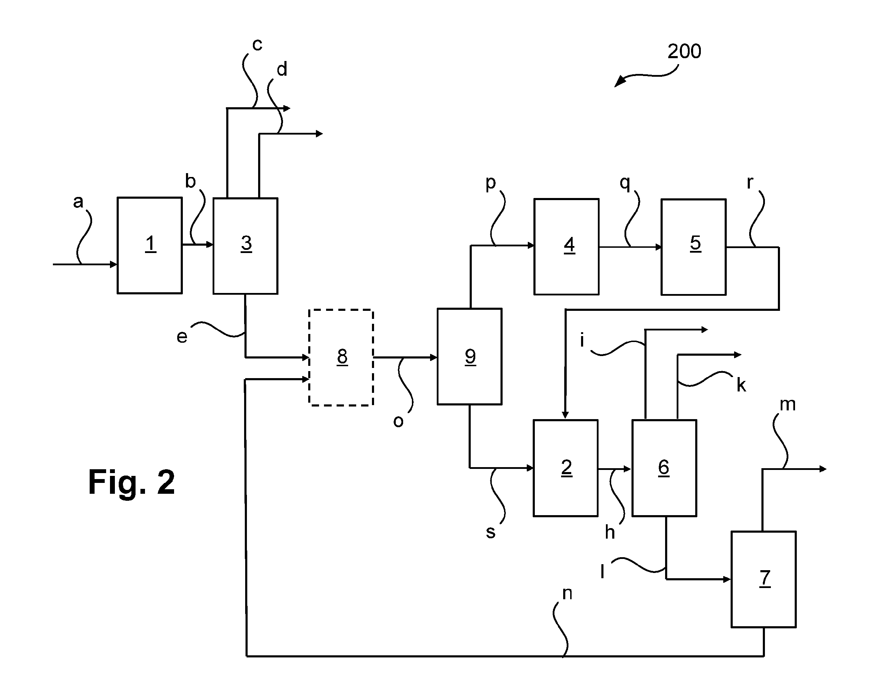

Je nach Zielsetzung kann in der Dampfspalteinheit 2 eine scharfe Spaltung (zur Ethylenmaximierung) oder milde Spaltung (zur Propylenmaximierung) erfolgen. Unabhängig von der Spaltschärfe wird jedoch tendenziell nur dann eine höhere Menge Butadien erzeugt, wenn der verwendete Einsatz (wie im Beispiel der Anlage 100 in Form des Dampfspalteinsatzstroms g nicht der Fall) noch ungesättigte Komponenten, nämlich insbesondere 1-Buten und/oder 2-Buten enthält. Ist eine erhöhte Butadienmenge erwünscht, kann eine Anlage 200 gemäß Figur 2 zum Einsatz kommen.

In Figur 2 ist eine Anlage gemäß einer weiteren Ausführungsform der Erfindung schematisch dargestellt und insgesamt mit 200 bezeichnet.

In der Anlage 200 wird ein Trennabstrom e der Trenneinheit 3 optional einer Hydroisomerisierungseinheit 8 zugeführt. Hierzu kann aus dem Trennabstrom e und dem zuvor erläuterten butadienarmen Reststrom n ein Sammelstrom gebildet werden, der hier auch als Hydroisomerisierungseinsatzstrom bezeichnet wird. Statt zur Hydroisomerisierungseinheit 8 kann der Trennabstrom e und der Reststrom n auch in Form eines Destillationseinsatzstroms o direkt zu einer Destillationseinheit 9 geführt werden, die Hydroisomerisierungseinheit 8 ist also optional. Wird eine Hydroisomerisierungseinheit 8 verwendet, wird in dieser das in dem Hydroisomerisierungseinsatzstrom bzw. dem Trennabstrom e und dem Reststrom n enthaltene 1-Buten zu 2-Buten umgesetzt, das in einem entsprechenden Hydroisomerisierungsabstrom bzw. dem aus diesem gebildeten Destillationseinsatzstrom o enthalten ist. Wie erläutert, wird auf diese Weise eine destillative Trennung in der Destillationseinheit 9 erleichtert.

In der Destillationseinheit 9 wird aus dem Destillationseinsatzstrom o durch Abtrennen von Butan und 2-Buten, bzw. des überwiegenden Anteils hiervon in Form des Stroms s ein Destillationsabstrom p gewonnen. Der Destillationsabstrom p enthält, sofern keine Hydroisomerisierungseinheit 8 vorgesehen ist, 1-Buten, ansonsten nicht. Ferner enthält der Destillationsabstrom p Isobuten und Isobutan. Der Destillationsabstrom p wird als Hydrierungseinsatzstrom einer Hydrierungseinheit, die hier, wie in Figur 1, mit 4 bezeichnet ist, zugeführt. In der Hydrierungseinheit wird, falls in dem Hydrierungseinsatzstrom p vorhanden, 1-Buten zu 1-Butan umgesetzt, ferner erfolgt eine Umsetzung von Isobuten zu Isobutan. Ein in der Hydrierungseinheit 4 erhaltener Hydrierungsabstrom enthält daher entweder eine Mischung aus im Wesentlichen n-Butan und Isobutan oder im Wesentlichen reines Isobutan. Der Hydrierungsabstrom wird anschließend als Skelettisomerisierungseinsatzstrom q einer Skelettisomerisierungseinheit, die auch hier mit 5 bezeichnet ist, zugeführt. Wiederum wird ein im Wesentlichen reiner n-Butanstrom als Skelettisomerisierungsabstrom erhalten und als Dampfspalteinsatzstrom r der Dampfspalteinheit 2 zugeführt.

In der Destillationseinheit 9 wird ferner, wie erläutert, ein im Wesentlichen Butan und 2-Buten enthaltender Strom erhalten, der ebenfalls als (weiterer) Dampfspalteinsatzstrom s einer Dampfspalteinheit, und hier einem gleichen oder unterschiedlichen Spaltofen wie der Dampfspalteinsatzstrom r, zugeführt werden kann.

Zu den Strömen a bis d sowie h bis n und den Einheiten 1 bis 3 sowie 6 und 7 sei auf die obigen Erläuterungen zu Figur 1 verwiesen.

## Patentansprüche

1. Verfahren zur Herstellung von Kohlenwasserstoffen, bei dem in einer Katalyseeinheit (1) unter Verwendung eines oder mehrerer, Oxygenate und/oder Olefine enthaltender Katalyseeinsatzströme (a) ein an n-Butan, Isobutan, 1-Buten, 2-Buten, Isobuten und Kohlenwasserstoffen mit mehr als vier und/oder weniger als drei Kohlenstoffatomen reicher Katalyseproduktstrom (b) erzeugt wird, und bei dem ferner in einer Dampfspalteinheit (2) unter Verwendung eines oder mehrerer Dampfspalteinsatzströme (g, r, s) ein Dampfspaltproduktstrom (h) erzeugt wird, **dadurch gekennzeichnet, dass** unter Verwendung des Katalyseproduktstroms (b) ein an 1-Buten, 2-Buten und Isobuten armer und zumindest Isobutan enthaltender Skelettisomerisierungseinsatzstrom (f, q) erzeugt wird, in welchem das Isobutan zumindest zum überwiegenden Teil durch Skelettisomerisieren zu n-Butan umgesetzt wird, und der danach zumindest zum Teil als der oder einer der Dampfspalteinsatzströme (g, r) verwendet wird.

2. Verfahren nach Anspruch 1, bei dem unter Verwendung zumindest eines Teils des Katalyseproduktstroms (b) ein Trenneinsatzstrom gebildet wird, aus dem die Kohlenwasserstoffe mit mehr als vier und/oder weniger als drei Kohlenstoffatomen unter Erhalt eines an n-Butan, Isobutan, 1-Buten, 2-Buten und Isobuten reichen Trennabstroms (e) zumindest zum überwiegenden Teil abgetrennt werden.

3. Verfahren nach Anspruch 2, bei dem unter Verwendung zumindest eines Teils des Trennabstroms (e) ein Hydrierungseinsatzstrom gebildet wird, in dem das 1-Buten, 2-Buten und Isobuten unter Erhalt eines Hydrierungsabstroms zumindest zum überwiegenden Teil durch Hydrieren zu n-Butan und Isobutan umgesetzt werden, wobei zumindest ein Teil des Hydrierungsabstroms bei der Bildung des Skelettisomerisierungseinsatzstroms (f) verwendet wird.

4. Verfahren nach Anspruch 2, bei dem unter Verwendung zumindest eines Teils des Trennabstroms (e) ein Destillationseinsatzstrom (o) gebildet wird, aus dem das n-Butan und 2-Buten unter Erhalt eines an n-Butan und 2-Buten armen Destillationsabstroms (p) zumindest zum überwiegenden Teil abgetrennt werden.

5. Verfahren nach Anspruch 4, bei den unter Verwendung zumindest eines Teils des Trennabstroms (e) ein Hydroisomerisierungseinsatzstrom gebildet wird, in dem das 1-Buten unter Erhalt eines Hydroisomerisierungsabstroms zumindest zum überwiegenden Teil durch Hydroisomerisieren zu 2-Buten umgesetzt wird, wobei zumindest ein Teil des Hydroisomerisierungsabstroms bei der Bildung des Destillationseinsatzstroms (o) verwendet wird.

6. Verfahren nach Anspruch 4 oder 5, bei dem unter Verwendung zumindest eines Teils des Destillationsabstroms (p) ein Hydrierungseinsatzstrom gebildet wird, in dem zumindest das Isobuten unter Erhalt eines Hydrierungsabstroms zumindest zum überwiegenden Teil durch Hydrieren zu Isobutan umgesetzt wird, wobei zumindest ein Teil des Hydrierungsabstroms bei der Bildung des Skelettisomerisierungseinsatzstroms (q) verwendet wird.

7. Verfahren nach einem der Ansprüche 4 bis 6, bei dem unter Verwendung zumindest eines Teils des aus dem Destillationseinsatzstrom (o) abgetrennten n-Butans und 2-Butens ein Strom gebildet wird, der als weiterer Dampfspalteinsatzstrom (s) verwendet wird.

8. Verfahren nach einem der vorstehenden Ansprüche, bei dem der Dampfspaltproduktstrom (h) Kohlenwasserstoffe mit vier Kohlenstoffatomen, darunter Butadien, sowie Kohlenwasserstoffe mit mehr als vier und/oder weniger als drei Kohlenstoffatomen enthält.

9. Verfahren nach Anspruch 8, bei dem unter Verwendung zumindest eines Teils des Dampfspaltproduktstroms (h) ein an Butadien und Kohlenwasserstoffen mit mehr als vier und/oder weniger als drei Kohlenstoffatomen armer Reststrom (n) gewonnen wird, der zumindest zum Teil bei der Erzeugung des Skelettisomerisierungseinsatzstroms (f, q) verwendet wird.

10. Anlage (100, 200) zur Herstellung von Kohlenwasserstoffen, mit einer Katalyseeinheit (1), die dazu eingerichtet ist, unter Verwendung eines oder mehrerer, Oxygenate und/oder Olefine enthaltender Katalyseeinsatzströme (a) einen an n-Butan, Isobutan, 1-Buten, 2-Buten, Isobuten und Kohlenwasserstoffen mit mehr als vier und/oder weniger als drei Kohlenstoffatomen reichen Katalyseproduktstrom (b) zu erzeugen, und mit einer Dampfspalteinheit (2), die dazu eingerichtet ist, unter Verwendung eines oder mehrerer Dampfspalteinsatzströme (g, r, s) einen Dampfspaltproduktstrom (h) zu erzeugen, **gekennzeichnet durch** Mittel, die dazu eingerichtet sind, unter Verwendung des Katalyseproduktstroms (b) einen an 1-Buten, 2-Buten und Isobuten armen und zumindest Isobutan enthaltenden Skelettisomerisierungseinsatzstrom (f, q) zu erzeugen, in diesem das Isobutan zumindest zum überwiegenden Teil **durch** Skelettisomerisieren zu n-Butan umzusetzen, und diesen danach zumindest zum Teil als den oder einen der Dampfspalteinsatzströme (g, r) zu verwenden.

11. Anlage (100, 200) nach Anspruch 10, die Mittel aufweist, die zur Durchführung eines Verfahrens nach einem der Ansprüche 1 bis 9 eingerichtet sind.
